Europäisches Patentamt

European Patent Office

Office européen des brevets

(19)

(11) EP 0 773 061 A1

(12) DEMANDE DE BREVET EUROPEEN

(43) Date de publication:
14.05.1997 Bulletin 1997/20

(51) Int Cl.6: **B01J 23/26**, B01J 35/10, C07C 17/20

(21) Numéro de dépôt: 96402318.8

(22) Date de dépôt: 31.10.1996

(84) Etats contractants désignés:
DE ES FR GB GR IT NL

(30) Priorité: 10.11.1995 FR 9513319

(71) Demandeur: ELF ATOCHEM S.A.
92800 Puteaux (FR)

(72) Inventeurs:
• Joubert, Philippe
69005 Lyon (FR)
• Garcia, François
69530 Brignais (FR)
• Lacroix, Eric
69480 Amberieux D'Azergues (FR)

(54) **Catalyseurs massiques à base d'oxyde de chrome, leur procédé de préparation et leur application à la fluoration d'hydrocarbures halogénés**

(57) L'invention a pour objet des catalyseurs massiques à base d'oxyde de chrome ou d'oxydes de chrome et éventuellement d'au moins un métal catalytiquement actif, présentant une surface spécifique supérieure à 100 m$^2$/g et un diamètre de pore médian supérieur à 5 nm.

L'invention concerne aussi un procédé de préparation desdits catalyseurs qui consiste essentiellement :

a) à former un précipité gélatineux aqueux d'hydroxyde de chrome ou d'hydroxydes de chrome et éventuellement d'au moins un autre métal catalytiquement actif

b) à substituer l'eau de ce précipité par un gaz à l'état de fluide supercritique pour former un solide poreux, et

c) calciner ledit solide.

L'invention a également pour objet l'application de ces catalyseurs à la fluoration par HF en phase gazeuse d'hydrocarbures halogénés, saturés ou oléfiniques.

Elle concerne aussi des catalyseurs partiellement ou totalement fluorés caractérisés en ce qu'ils présentent une surface spécifique supérieure à 25 m$^2$/g et un diamètre de pore médian supérieur à 9 nm.

EP 0 773 061 A1

Printed by Jouve, 75001 PARIS (FR)

## Description

La présente invention a trait au domaine de la fluoration des hydrocarbures halogénés par catalyse en phase gazeuse. Elle concerne plus particulièrement des catalyseurs massiques, fluorés ou non, à base d'oxyde de chrome ou d'oxydes de chrome et éventuellement d'au moins un autre métal catalytiquement actif, leur procédé de préparation et leur application à la synthèse d'hydrohaloalcanes.

Les composés métalliques (par exemple chrome, fer, cobalt, nickel, vanadium, zinc, cuivre, manganèse, magnésium ...) sont connus pour leur effet catalytique dans les réactions de fluoration des hydrocarbures halogénés. A ce titre, les dérivés à base de chrome, notamment de trifluorure de chrome hydraté ou d'oxyde de chrome hydraté, sont particulièrement recommandés (voir les brevets US 2745 886 et US 3 426 009).

Les catalyseurs de fluoration proposés dans la littérature sont soit supportés (principalement sur du charbon ou de l'alumine), soit massiques.

Les catalyseurs massiques à base de chrome sont élaborés à partir de matières premières variées.

Ainsi, dans EP 313061, on prépare un catalyseur à base d'oxyde de chrome par pyrolyse de bichromate d'ammonium à une température comprise entre 500 et 700°C.

Le brevet FR 2 501 062 propose des microbilles d'oxyde de chrome amorphe obtenues par gélification d'un sol d'hydroxyde de chrome dans un solvant non miscible ou partiellement miscible à l'eau et séchage.

Dans EP 514 932, on décrit la préparation d'un catalyseur à base d'oxyde de chrome amorphe à partir d'un précipité d'hydroxyde de chrome, lequel précipité est obtenu par mélange d'un sel de chrome et d'ammoniaque. Le produit obtenu après séchage (70-200°C) et calcination (380-460°C) présente une surface spécifique d'au moins 170 m²/g.

Le brevet US 4 912 270 propose un aérogel à base d'oxyde ou d'hydroxyde de chrome par réduction du trioxyde de chrome au moyen d'un alcool et élimination dudit alcool en conditions supercritiques. L'aérogel obtenu présente une surface spécifique supérieure à 400 m²/g, un volume poreux au moins égal à 2 cm³/g et une distribution homogène des pores (porosimétrie au mercure).

L'ajout de "dopants" dans les catalyseurs au chrome a été préconisé pour améliorer les performances catalytiques et notamment limiter les réactions secondaires telles que l'oxydation de l'acide chlorhydrique -susceptible de générer du chlore réagissant avec les hydrocarbures halogénés.

Ainsi, dans EP 546 883, on propose un catalyseur à base d'oxydes de chrome et de nickel obtenu par gélification d'un sol d'hydroxydes de chrome et de nickel, séchage et calcination à une température comprise entre 250 et 450°C. Le produit obtenu présente un rapport atomique Ni/Cr compris entre 0,05 et 5.

Dans US 4 547 483, on prépare un catalyseur à base de chrome et de magnésium par séchage (20-500°C) d'une pâte résultant du mélange en milieu aqueux d'un sel de chrome et d'oxyde ou d'hydroxyde de magnésium.

Enfin, dans EP 502 605, on décrit un catalyseur au chrome et zinc obtenu soit par imprégnation d'oxyde de chrome par un composé du zinc (halogénure, nitrate ou carbonate), soit par co-précipitation des hydroxydes de chrome et de zinc ou encore par mélange et broyage de zinc insoluble et du catalyseur au chrome.

Les dérivés oxygénés du chrome de structure amorphe réagissent avec les agents fluorants, et plus particulièrement avec l'acide fluorhydrique. La vitesse de fluoration de ces composés décroît exponentiellement en fonction du taux de fluoration du catalyseur.

Dans les conditions utilisées généralement en phase gazeuse (T < 450°C), la fluoration se décompose en deux étapes :

- une première fluoration, rapide, qui permet d'atteindre en quelques heures un rapport atomique F/Cr de l'ordre de 1,5 (taux de fluoration : 50 %),
- et une seconde étape, nettement plus longues (plusieurs centaines d'heures), qui conduit à un rapport atomique F/Cr proche de 3.

La fluoration du catalyseur étant exothermique, il est nécessaire de conduire la réaction de manière très progressive (dilution dans un inerte, augmentation lente et contrôlée de la température). En outre, la réaction produit de l'eau qui peut, d'une part inhiber l'activité catalytique et, d'autre part, provoquer, en présence d'hydracides, une corrosion de l'outil industriel.

Pour ces raisons, on préfère généralement mener l'étape rapide de fluoration en l'absence de produit organique susceptible de réagir avec l'agent fluorant.

Le solide partiellement fluoré obtenu après cette étape dite "d'activation du catalyseur" catalyse déjà les réactions de fluoration des hydrocarbures halogénés. En raison de la durée trop longue, l'étape de fluoration lente a souvent lieu, industriellement, dans l'unité de fluoration du produit organique en présence du mélange réactionnel (organiques, HF, HCl, ...).

La perfluoration du catalyseur au sein du réacteur industriel présente des inconvénients :

- elle nécessite de démarrer l'unité industrielle de manière progressive afin d'étaler dans le temps la production d'eau et de calories due à la fluoration du catalyseur,

- elle présente des risques d'emballement de la température avec dégradation de l'outil industriel et des propriétés mécaniques du catalyseur,

- enfin, elle peut conduire ponctuellement à des quantités d'eau importantes, non souhaitées du point de vue de la corrosion.

Afin de limiter la production d'eau et de calories dans la phase lente de la réaction de fluoration, plusieurs solutions sont proposées.

Une première solution consiste à employer des catalyseurs au chrome présentant une très faible réactivité vis-à-vis de l'agent de fluoration, ce qui permet ainsi de réduire de manière sensible, voire de supprimer, l'étape d'activation du catalyseur. De tels catalyseurs fortement cristallisés sont par exemple décrits dans la demande de brevet EP 657408 au nom de la Demanderesse.

Une deuxième solution préconise l'utilisation de catalyseurs au chrome amorphe présentant une importante réactivité au regard de l'agent fluorant.

Ce type de catalyseurs est notamment proposé par GRANIER et al. (Bulletin de la Société Chimique de France, N° 3-4, pp. I.91-I.96, 1984). Les auteurs ont montré que les pores de diamètre compris entre 2 et 10 nm jouent un rôle prépondérant dans la réaction de fluoration alors que les pores plus petits ne sont le siège que de réactions irréversibles entre les atomes de chrome et de fluor (ce type d'atome de fluor n'intervenant pas directement lors de la réaction de fluoration des hydrocarbures halogénés).

Il a maintenant été trouvé que le séchage de catalyseurs à base d'oxyde de chrome amorphe au moyen d'un fluide supercritique permet d'obtenir des catalyseurs à réactivité améliorée. De tels catalyseurs, tout en gardant les avantages d'un catalyseur massique, possèdent un taux de fluoration lors de l'étape d'activation nettement amélioré et par là-même permettent de limiter la formation d'eau dans le réacteur industriel.

L'invention a donc pour objet des catalyseurs massiques à base d'oxyde de chrome ou d'oxydes de chrome et éventuellement d'au moins un autre métal catalytiquement actif, obtenus par un procédé qui consiste essentiellement :

a) à former un précipité gélatineux aqueux d'hydroxyde de chrome ou d'hydroxydes de chrome et éventuellement d'au moins un autre métal catalytiquement actif, et

b) à substituer l'eau de ce précipité par un gaz à l'état de fluide supercritique pour former un solide poreux, et

c) calciner ledit solide.

L'invention a également pour objet l'application de ces catalyseurs à la fluoration par HF en phase gazeuse d'hydrocarbures halogénés, saturés ou oléfiniques.

Dans les catalyseurs selon l'invention, la surface spécifique est supérieure à 100 $m^2$/g et de préférence 150 $m^2$/g et le diamètre médian de la distribution des volumes poreux mesuré selon la méthode par adsorption de l'azote est supérieur à 5 nm.

Parmi ces catalyseurs, l'invention concerne plus particulièrement ceux dont le volume poreux est supérieur à 0,15 $g/cm^3$ et de préférence 0,18 $g/cm^3$ (méthode par adsorption d'azote; diamètre des pores compris entre 2 et 200 nm).

Dans les catalyseurs selon l'invention, le rapport atomique : autre métal actif/chrome est généralement inférieur à 1,2 et de préférence compris entre 0,1 et 1.

Le précipité gélatineux du procédé selon l'invention peut être obtenu de façon connue en soi.

On peut par exemple former le précipité par dissolution dans l'eau d'un sel de chrome et d'un sel d'au moins un autre métal, et gélification au moyen d'une base.

Comme sel de chrome, on peut citer les chlorures, nitrates, sulfates, fluorures, perchlorates, acétates, acétylacétonates de chrome. On préfère les sulfates, les chlorures, les nitrates et les acétates.

Le sel de métal autre que le chrome est généralement choisi parmi les sels de même nature que ceux précités dont le métal appartient au groupe formé par Mg, Ni, Zn, Fe, Co, V, Mn, Cu et Al. De préférence, on utilise les nitrates ou les chlorures de Ni.

Comme base, on peut citer NaOH, KOH, Ca(OH)$_2$, Na$_2$CO$_3$, NaHCO$_3$, (NH$_4$)$_2$CO$_3$, NH$_4$HCO$_3$ et les amines.

On peut également former le précipité gélatineux à partir d'un sol d'hydroxyde de chrome et d'hydroxyde d'au moins un autre métal, selon les méthodes connues de l'homme du métier, par exemple par peptisation ou par évaporation d'une partie du solvant.

Comme hydroxyde de métal autre que le chrome, on peut citer les hydroxydes de Mg, Ni, Zn, Fe, Co, V, Mn, Cu et Al et de préférence de nickel.

Divers additifs peuvent aussi être ajoutés durant la préparation du précipité gélatineux pour parfaire les propriétés physico-chimiques et catalytiques du catalyseur final. On peut ainsi ajouter (% par rapport au poids du catalyseur final) :

- 1 à 80 % d'Al$_2$O$_3$, xH$_2$O pour améliorer la tenue mécanique du catalyseur final,
- 0,1 à 5 % de graphite pour faciliter la mise en forme par pastillage ou par extrusion,
- 0,1 à 20 % de floculant tel que les polyacrylates ou polyacrylamides pour faciliter la filtration du gâteau récupéré après neutralisation.

A partir du précipité gélatineux d'hydroxyde de chrome ou d'hydroxydes de chrome et éventuellement d'au moins un autre métal catalytiquement actif, on prépare le catalyseur selon l'invention en séchant le précipité dans un dispositif tel que présenté dans la Figure 1.

Ce dispositif est constitué d'une enceinte de séchage [1] chauffée par un four [2]. L'enceinte est alimentée en fluide supercritique au moyen du tube [3] relié au réservoir [4] et équipée du tube d'évacuation [5] du fluide de percolation.

En conditions de fonctionnement, on introduit le précipité gélatineux aqueux à sécher dans l'enceinte [1]. On soutire le gaz liquéfié contenu dans le réservoir [4] équipé du tube plongeur [6] au moyen de la pompe [8]. En général, le gaz liquéfié est préalablement refroidi avant le pompage par contact avec l'échangeur [9]. Le gaz liquéfié issu de la pompe est réchauffé au moyen de l'échangeur [10] et introduit dans l'enceinte [1] par le tube [3]. Dans l'enceinte [1] mise sous pression au moyen de la vanne [7] et chauffée par le four [2], la pression et la température sont telles que le fluide circulant en continu se trouve à l'état supercritique. Le fluide contenant l'eau extraite du précipité gélatineux est évacué en continu par le tube [5].

Après un temps de fonctionnement pouvant varier de l'ordre d'une heure à plusieurs dizaines d'heures, on arrête l'alimentation en gaz et on procède à la dépressurisation de l'enceinte [1]. On récupère un solide poreux séché.

Lors du séchage, le précipité gélatineux est avantageusement mis en forme suivant les techniques connues, par exemple par extrusion.

Le gaz mis en oeuvre dans le dispositif est choisi parmi les gaz présentant une pression critique inférieure à 20 MPa, et de préférence 8 MPa, et une température critique n'excédant pas 200°C, et de préférence 160°C. On utilise avantageusement CO$_2$, SO$_2$, CH$_3$Cl, C$_2$H$_5$Cl et les FORANES® notamment le chlorodifluorométhane, le trifluorométhane et le tétrafluoroéthane, ainsi que les mélanges de ces gaz.

La température de l'enceinte est supérieure à la température critique du gaz utilisé, laquelle température dépend bien évidemment de la nature de ce gaz. A titre d'illustration, lorsque on utilise le gaz carbonique, on chauffe le four à une température supérieure à 30°C et de préférence supérieure à 80°C.

La température de l'échangeur [9] est généralement comprise entre -5 et +15°C.

La température de l'échangeur [10] est généralement supérieure à 31°C et de préférence comprise entre 80 et 120°C.

La pression appliquée dans l'enceinte du dispositif est supérieure à la pression critique du gaz utilisé, laquelle pression dépend de la nature de ce gaz. Généralement, la pression est supérieure à 8 MPa et de préférence comprise entre 20 et 40 MPa.

Après un temps de fonctionnement pouvant varier de l'ordre d'une heure à plusieurs dizaines d'heures, on récupère un solide poreux à base d'oxyde de chrome ou d'oxydes de chrome et éventuellement d'au moins un autre métal catalytiquement actif.

Le solide poreux peut être mis en forme de façon connue en soi, par exemple par pastillage, extrusion ou granulation. Afin de faciliter la mise en forme par pastillage ou extrusion, il est avantageux d'ajouter une quantité de graphite telle que la teneur varie de 0,1 à 5 % par rapport au poids du catalyseur final.

L'étape c) de calcination du solide poreux est généralement réalisée sous atmosphère inerte, par exemple sous azote, à une température comprise entre 200 et 500°C.

Les catalyseurs massiques selon l'invention peuvent être utilisés pour la fluoration par HF en phase gazeuse d'hydrocarbures halogénés, saturés ou oléfiniques. Ils conviennent particulièrement bien à la fluoration d'hydrocarbures halogénés conduisant à des composés fluorés en C$_1$ à C$_5$ contenant un ou plusieurs atomes d'hydrogène. Comme exemples d'hydrocarbures halogénés de départ, on peut citer, à titre non limitatif, les composés suivants : CHCl$_3$, CH$_2$Cl$_2$, CH$_2$ClF, CCl$_2$=CHCl, CH$_3$-CHCl$_2$, CH$_2$Cl-CH$_2$Cl, CH$_2$=CHCl, CHCl$_2$-CClF$_2$, CHCl$_2$-CF$_3$, CH$_2$F-CClF$_2$, CHClF-CF$_3$, CHF$_2$-CClF$_2$, CH$_2$Cl-CF$_3$, CCl$_3$-CH$_3$, CCl$_2$F-CH$_3$, CClF$_2$-CH$_3$, CH$_3$-CCl$_2$-CH$_3$, CCl$_3$-CF$_2$-CH$_3$, CCl$_3$-CF$_2$-CHCl$_2$, CCl$_3$-CF$_2$-CH$_2$Cl, CHCl$_2$-CHCl-CH$_3$, CH$_2$Cl-CHCl-CH$_3$ ainsi que CCl$_2$=CCl$_2$; ce dernier composé ne contient pas d'hydrogène, mais l'addition d'HF conduit à des composés hydrohalogénés.

Pour travailler à l'activité optimum, le catalyseur nécessite un traitement à l'acide fluorhydrique, dilué ou non avec de l'azote. Il est généralement recommandé de contrôler l'exothermicité de l'activation en jouant sur l'ajout d'un diluant de HF et en commençant ce traitement à basse température (150-250°C). Après que les pics d'adsorption de l'HF sont passés, on peut monter progressivement la température pour atteindre un maximum de 350-450°C en fin d'activation.

De tels catalyseurs partiellement ou totalement fluorés constituent également un objet de la présente invention. Ils sont caractérisés en ce qu'ils présentent une surface spécifique supérieure à 25 m$^2$/g, de préférence à 30 m$^2$/g, et

un diamètre de pore médian supérieur à 9 nm, de préférence à 10 nm.

En outre, ils présentent un volume poreux supérieur à 0,08 cm$^3$/g et de préférence à 0,1 cm$^3$/g.

La température de la fluoration dépend de la réaction étudiée et bien évidemment des produits de réaction désirés. Ainsi, pour une addition d'HF sur une double liaison ou une substitution partielle des atomes de chlore par le fluor, on travaille à des températures comprises entre 50 et 350°C. La substitution totale des atomes de chlore nécessite généralement des températures comprises entre 300 et 500°C.

Le temps de contact dépend également de la réaction étudiée et des produits recherchés. Il varie généralement dans de larges limites entre 0,1 et 100 secondes. Industriellement, un bon compromis entre taux de conversion et productivité est obtenu pour des temps de contact inférieurs à 30 secondes.

Le rapport molaire HF/composé organique est également lié à la réaction étudiée. Il dépend entre autres de la stoechiométrie de la réaction. Dans la majorité des cas, ils peut varier entre 1/1 et 20/1, mais, là aussi, afin d'obtenir des productivités élevées, il est souvent inférieur à 10.

La pression de travail est de préférence comprise entre 1 et 20 bars absolus (0,1 à 2 MPa).

Les catalyseurs selon l'invention peuvent, selon leur résistance mécanique, travailler en lit fixe ou en lit fluidisé.

Les catalyseurs dont l'activité a chuté par suite d'un encrassement, peuvent être régénérés par balayage du catalyseur avec un composé susceptible d'oxyder et de transformer les produits (organiques, coke, ...) déposés sur le catalyseur en produits volatils. A ce titre, l'oxygène ou un mélange contenant de l'oxygène (air par exemple) convient parfaitement et permet de restaurer l'activité initiale du catalyseur. Il est bien sûr nécessaire de contrôler l'exothermie de cette "combustion" en contrôlant le débit d'oxygène (en début de régénération, dilution importante dans un inerte) pour prévenir un emballement de cette régénération et éviter de dépasser des températures supérieures à 600°C.

Pour maintenir l'activité du catalyseur, il est également possible d'effectuer la réaction de fluoration en présence d'oxygène et/ou de chlore introduit(s) dans un rapport molaire oxydant/composé organique pouvant aller de 0,001 à 0,05 et, de préférence, compris entre 0,005 et 0,03.

Les exemples suivants illustrent l'invention sans toutefois la limiter.

Dans les exemples, le solide minéral poreux est caractérisé selon les méthodes suivantes :

La perte de masse est calculée à partir des pesées effectuées avant et après le séchage.

La surface spécifique S$_{BET}$ est mesurée par la méthode conventionnelle selon Brunauer Emmet Teller.

Le volume poreux Vp$_{N2}$ est mesuré par la détermination de la quantité d'azote condensé dans les pores selon la méthode BJH (J. Am. Chem. Soc. vol. 73, p. 373, 1951).

Le volume poreux Vp$_{Hg}$ est mesuré par intrusion de mercure sous pression selon la méthode de Washburn.

La surface spécifique Sp$_{Hg}$ est calculée à partir des données expérimentales d'intrusion de mercure sous pression en faisant l'hypothèse de pores cylindriques, selon la formule :

$$Sp_{Hg} = 4x10^3 \; \Sigma_i \; Vi/Di$$

dans laquelle

Sp$_{Hg}$ est la surface totale des pores exprimée en m$^2$/g
Vi est le volume du pore i exprimé en cm$^3$/g
Di est le diamètre du pore i exprimé en nm.

**PREPARATION DES CATALYSEURS**

**EXEMPLE 1** *catalyseurs A et B*

On prépare deux lots identiques de catalyseurs A et B dans les conditions suivantes.

On dissout 100 g (0,25 mole) de Cr(NO$_3$)$_3$.9H$_2$O et 59,5 g de NiCl$_2$.6H$_2$O dans 500 ml d'eau à 80°C sous agitation (300 RPM) pendant 2 heures.

Après refroidissement, on ajoute à la solution ainsi obtenue 90 ml d'une solution ammoniacale (28 % en poids de NH$_3$) à raison de 2 ml/min.

Après décantation et élimination du surnageant, on récupère un précipité gélatineux qui est lavé quatre fois avec 250 ml d'eau.

Le précipité gélatineux obtenu (144,4g) est mis sous forme d'extrudats (diamètre : 3 mm ; longueur: 4-8 mm) qui sont placés dans le panier grillagé de l'enceinte de séchage (diamètre : 35 mm ; longueur: 120 mm) du dispositif de la figure 1.

Le séchage est réalisé au moyen de CO$_2$ (débit: 2,8 kg/h) à 110°C, sous pression (26 MPa) pendant 5,5 heures.

Les extrudats sont calcinés sous balayage permanent d'azote dans les conditions suivantes :

- chauffage jusqu'à 200°C (100°C/h),
- palier à 200°C pendant 2 h,
- chauffage jusqu'à 350°C (100°C/h),
- palier à 350°C pendant 4 h,
- refroidissement.

Les caractéristiques physico-chimiques des extrudats obtenus après séchage et des catalyseurs A et B (après calcination) sont présentées dans le tableau 1.

Les courbes cumulées des volumes poreux mesurés par adsorption/désorption d'azote sont présentées dans la figure 2.

**EXEMPLE 2 (comparatif)** catalyseur C

Les extrudats non séchés obtenus selon l'exemple 1 sont séchés dans une étuve à vide à 50°C sous une pression de 20 kPa pendant 12 heures, puis calcinés dans les conditions de l'exemple 1.

Les caractéristiques physico-chimiques des extrudats obtenus après séchage et du catalyseur C (après calcination) sont présentées dans le tableau 1.

La courbe cumulée des volumes poreux mesurés par adsorption/désorption d'azote est présentée dans la figure 2.

**EXEMPLES DE FLUORATION**

Dans les exemples suivants :

- les pourcentages indiqués sont des pourcentages molaires,
- l'acide fluorhydrique utilisé est un produit commercial ne contenant que des traces d'eau,
- le chloro-1 trifluoro-2,2,2 éthane (F133a) de départ est un produit pur à 99,9 %,
- le réacteur utilisé est un tube en Inconel de 20 cm$^3$ chauffé par l'intermédiaire d'un four tubulaire.

L'activation du catalyseur par HF est réalisée dans ce réacteur sur un échantillon de 10 cm$^3$. Après un séchage sous azote (5 l/h) pendant 2 heures à 250°C, l'acide fluorhydrique dilué par de l'azote (rapport molaire $N_2$/HF = 2/1) est additionné progressivement à cette même température.

Après passage des pics d'exothermicité, on porte la température à 350°C et le débit d'HF à 0,2 mole/heure durant 10 heures.

Avant leur introduction dans le réacteur, les réactifs sont mélangés et chauffés à la température de réaction dans un préchauffeur en Inconel.

Après lavage à l'eau -afin d'éliminer les hydracides- et séchage sur $CaCl_2$, les produits de réaction sont analysés en ligne, par chromatographie en phase gazeuse.

Les caractéristiques des catalyseurs A, B et C après l'étape d'activation (catalyseur activé) sont présentées dans le tableau 1.

**EXEMPLES 3 ET 4**

On effectue les tests de fluoration du F133a sous pression atmosphérique, en absence d'oxygène avec les catalyseurs A, B et C. Les résultats de fluoration sont résumés dans le tableau 2.

Dans l'exemple 3, le catalyseur B a fonctionné 28 heures à 350°C puis 24 heures à 320°C.

Pour chaque catalyseur A, B et C récupéré à l'issue des tests de fluoration (catalyseur usagé), on mesure le taux de fluoration (tableau 1) et la courbe de distribution du volume poreux cumulé en fonction du diamètre des pores (figure 3).

## TABLEAU 1

| EXEMPLE | Etat | Porosité Hg | | | | Porosité N$_2$ | | | Taux de fluoration (%) [**] |
| | | 8 nm<D<126 μm | | 8 nm<D<2 μm | | 2 nm<D<200 nm | | | |
| | | Vp (cm$^3$/g) | Sp (m²/g) | Vp (cm$^3$/g) | Sp (m²/g) | S(BET) (m²/g) | Sp * (m²/g) | VpN$_2$ (cm$^3$/g) | |
|---|---|---|---|---|---|---|---|---|---|
| A | Extrudats séchés | 0,288 | 18,8 | 0,222 | 18,7 | | 75 | 0,115 | |
| | Catalyseur | 0,434 | 45,6 | 0,353 | 45,5 | 106 | 139 | 0,212 | |
| 1 | Catalyseur activé | | | | | 71,9 | 101,6 | 0,202 | 38,9 |
| | Catalyseur usagé | | | | | 45,2 | 62,1 | 0,143 | 89,8 |
| B | Extrudats séchés | 0,299 | 18,5 | 0,231 | 18,4 | 82 | 60 | 0,099 | |
| | Catalyseur | 0,384 | 41 | 0,324 | 41 | 106 | 134 | 0,226 | |
| | Catalyseur activé | | | | | 67,8 | 94,6 | 0,202 | 40,7 |
| | Catalyseur usagé | | | | | 40 | 55,6 | 0,142 | 82,3 |
| 2  C (comparatif) | Extrudats séchés | <0,09 | <4 | <0,019 | <4 | | | | |
| | Catalyseur | 0,06 | 9,2 | 0,038 | 9,2 | 107 | 140 | 0,133 | |
| | Catalyseur activé | | | | | | | | 31,6 |
| | Catalyseur usagé | | | | | 25,7 | 43,7 | 0,066 | 58,1 |

Sp* : Surface des pores de diamètre D calculée par la méthode BJH à partir des données de désorption d'azote.

$$^{**} : \text{taux de fluoration} = \frac{(\% \text{ F masse}) / 19}{(\% \text{ masse Ni}) \times 2/58,7 + (\% \text{ masse Cr}) \times 3/52} \times 100$$

EP 0 773 061 A1

## TABLEAU 2

| CATALYSEUR | EXEMPLE 3 | | | | EXEMPLE 4 (comparatif) | |
|---|---|---|---|---|---|---|
| | A | | B | | C | |
| Age du catalyseur (heures) | 28 | 96 | 28 | 52 | 24 | 48 |
| Rapport molaire HF/F133a | 4,1 | 4 | 4 | 4 | 3,7 | 3,8 |
| Temps de contact (seconde) | 0,5 | 0,5 | 0,5 | 0,5 | 0,5 | 0,5 |
| Température (°C) | 350 | 350 | 350 | 320 | 350 | 350 |
| Taux de transformation du F133a (%) | 17,7 | 16,9 | 16,8 | 7,1 | 10,4 | 10,2 |
| Sélectivité F134a ($CF_3$-$CH_2F$) (%) | 98,1 | 98,2 | 98,2 | 98,9 | 98,1 | 98,1 |
| Sélectivité F1122 ($CF_2$=CHCl) (%) | 1,3 | 1,4 | 1,4 | 1,0 | 1,5 | 1,7 |
| Sélectivité série F120 (*) (%) | 0,3 | 0,1 | 0,1 | 0,1 | 0,4 | 0,2 |
| Sélectivité F143a ($CF_3$-$CH_3$) (%) | 0,1 | 0,2 | 0,2 | traces | traces | traces |
| Sélectivité (autres) | 0,2 | 0,1 | 0,1 | traces | traces | traces |

(*) série F120 : ensemble des haloéthanes saturés ayant un seul atome d'hydrogène.

EP 0 773 061 A1

**Revendications**

1. Catalyseurs massiques à base d'oxyde de chrome ou d'oxydes de chrome et éventuellement d'au moins un autre métal catalytiquement actif, caractérisés en ce qu'ils présentent une surface spécifique supérieure à 100 m$^2$/g et un diamètre de pore médian supérieur à 5 nm.

2. Catalyseurs selon la revendication 1 caractérisés en ce que la surface est supérieure à 150 m$^2$/g.

3. Catalyseurs selon la revendication 1 ou 2 caractérisés en ce qu'ils présentent un volume poreux supérieur à 0,15 cm3/g.

4. Catalyseurs selon l'une des revendications 1 à 3 caractérisés en ce que le métal catalytiquement actif est choisi parmi Mg, Ni, Zn, Fe, Co, V, Mn, Cu et Al.

5. Catalyseurs selon l'une des revendications 1 à 4 caractérisés en ce qu'ils se présentent sous forme de pastilles, d'extrudats, de granulés ou de billes.

6. Catalyseurs selon l'une des revendications 1 à 4 caractérisés en ce que le rapport atomique : autre métal actif/ chrome est inférieur à 1,2.

7. Catalyseurs selon la revendication 6 caractérisés en ce que le rapport atomique : autre métal actif/chrome est compris entre 0,1 et 1.

8. Procédé de préparation des catalyseurs selon l'une des revendications 1 à 7 caractérisé en ce qu'il consiste essentiellement :

   a) à former un précipité gélatineux aqueux d'hydroxyde de chrome ou d'hydroxydes de chrome et d'au moins un autre métal catalytiquement actif, et
   b) à substituer l'eau de ce précipité par un gaz à l'état de fluide supercritique pour former un solide poreux, et
   c) calciner ledit solide.

9. Procédé selon la revendication 8 caractérisé en ce que le précipité gélatineux est formé par dissolution dans l'eau d'un sel de chrome choisi parmi les chlorures, nitrates, sulfates, fluorures, perchlorates, acétates, acétylacétonates de chrome et d'un sel d'au moins un autre métal choisi parmi le magnésium, nickel, zinc, fer, cobalt, vanadium, manganèse, cuivre et aluminium, et gélification au moyen d'une base.

10. Procédé selon la revendication 8 caractérisé en ce que le précipité gélatineux est formé par peptisation ou évaporation d'une partie du solvant d'un sol d'hydroxyde de chrome et d'hydroxyde d'au moins un autre métal choisi parmi le magnésium, nickel, zinc, fer, cobalt, vanadium, manganèse, cuivre et aluminium.

11. Procédé selon l'une des revendications 8 à 10 caractérisé en ce que le gaz est choisi parmi les gaz présentant une pression critique inférieure à 20 MPa et une température critique n'excédant pas 200°C.

12. Procédé selon la revendication 11 caractérisé en ce que le gaz est CO$_2$, SO$_2$, CH$_3$Cl, C$_2$H$_5$Cl et les FORANES® ainsi que les mélanges de ces gaz.

13. Procédé selon la revendication 12 caractérisé en ce que les FORANES® sont choisis parmi le chlorodifluorométhane, le trifluorométhane et le tétrafluoroéthane.

14. Procédé selon l'une des revendications 8 à 13 caractérisé en ce la substitution de l'eau du précipité au moyen du fluide supercritique est effectuée en continu.

15. Procédé selon l'une des revendications 8 à 14 caractérisé en ce que la température de calcination est comprise entre 200 et 500°C.

16. Procédé de fluoration par HF en phase gazeuse d'hydrocarbures halogénés, saturés ou oléfiniques, caractérisé en ce que l'on utilise un catalyseur selon l'une des revendications 1 à 7.

**17.** Procédé selon la revendication 16 caractérisé en ce que l'hydrocarbure halogéné est choisi parmi les composés fluorés en $C_1$ à $C_5$.

**18.** Procédé selon la revendication 17 caractérisé en ce que l'hydrocarbure halogéné est le chloro-1 trifluoro-2,2,2 éthane (F133a).

**19.** Catalyseurs massiques à base d'oxyde de chrome ou d'oxydes de chrome et éventuellement d'au moins un autre métal catalytiquement actif, partiellement ou totalement fluorés, caractérisés en ce qu'ils présentent une surface spécifique supérieure à 25 $m^2$/g et un diamètre de pore médian supérieur à 9 nm.

**20.** Catalyseurs selon la revendication 19 caractérisés en ce que la surface est supérieure à 30 $m^2$/g.

**21.** Catalyseurs selon la revendication 19 ou 20 caractérisés en ce qu'ils présentent un volume poreux supérieur à 0,08 cm3/g.

EP 0 773 061 A1

Office européen
des brevets

**RAPPORT DE RECHERCHE EUROPEENNE**

Numero de la demande
EP 96 40 2318

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | CH-A-682 134 (LONZA AG) 30 Juillet 1993<br><br>* revendications 1,4 *<br>--- | 1,2,19,20 | B01J23/26<br>B01J35/10<br>C07C17/20 |
| D,X | EP-A-0 514 932 (DAIKIN IND LTD) 25 Novembre 1992<br>* revendications 1-10 *<br>--- | 1,2,5,16-20 | |
| P,X | US-A-5 475 167 (M. J. NAPPA)<br><br>* revendications 1,4,5 *<br>* colonne 2, ligne 24 - ligne 44 *<br>--- | 1,2,18,19 | |
| X | EP-A-0 546 883 (ELF ATOCHEM)<br><br>* exemples 1-15 *<br>* revendication 1 *<br>--- | 1-10,15-21 | |
| A | EP-A-0 641 598 (SHOWA DENKO KK) 8 Mars 1995<br>* page 4, ligne 54 - page 5, ligne 5 *<br>--- | 4,6,7 | DOMAINES TECHNIQUES RECHERCHES (Int.Cl.6) |
| A | EP-A-0 055 958 (ATOCHEM) 14 Juillet 1982<br>--- | | B01J<br>C07C |
| A | EP-A-0 055 652 (ATOCHEM) 7 Juillet 1982<br>----- | | |

Le présent rapport a été établi pour toutes les revendications

| Lieu de la recherche | Date d'achèvement de la recherche | Examinateur |
|---|---|---|
| LA HAYE | 22 Janvier 1997 | Thion, M |